# EUROPEAN PATENT SPECIFICATION

(11) **EP 2 304 077 B1**
(45) Date of publication and mention of the grant of the patent: **16.05.2012**
(21) Application number: 09773976.7
(22) Date of filing: 27.05.2009
(51) Int. Cl.: A61L 29/14

(54) **METHODS FOR MAKING ANTIMICROBIAL COATINGS**
VERFAHREN ZUR HERSTELLUNG ANTIMIKROBIELLER BESCHICHTUNGEN
PROCÉDÉS DE FABRICATION DE REVÊTEMENTS ANTIMICROBIENS

(30) Priority: 30.06.2008 US 164414
(43) Date of publication of application: 06.04.2011
(73) Proprietor: Baxter International Inc., Deerfield, Illinois 60015-4633 (US); Baxter Healthcare SA, 8152 Glattpark (Opfikon) (CH)
(72) Inventor: KRONGAUZ, Vadim, V., Bartlett IL 60103 (US)
(74) Representative: Dee, Ian Mark
(86) International application number: PCT/US2009/045278
(87) International publication number: WO 2010/002524

(56) References cited:
- WO-A1-2007/000590
- WO-A2-2008/036377
- US-A- 5 718 694
- US-A- 6 106 505
- KIM ET AL: "Antimicrobial effects of silver nanoparticles" NANOMEDICINE: NANOTECHNOLOGY, BIOLOGY AND MEDICINE, ELSEVIER, NL, vol. 3, no. 1, 28 March 2007 (2007-03-28), pages 95-101, XP005928990 ISSN: 1549-9634

## Description

### BACKGROUND

### Field of the Disclosure

The disclosure relates generally to antimicrobial coating compositions and methods for making and processing such coatings. More particularly, the disclosure is directed to methods of making antimicrobial coating compositions comprising transition metals, methods for forming such coatings on substrates, such as medical devices, and methods for processing such coatings.

### Brief Description of Related Technology

Even brief exposure to surfaces having microbial contamination can introduce bacterial, viral, fungal, or other undesirable infections to humans and animals. Of particular concern is preventing or reducing microbial infection associated with the use of invasive medical devices such as catheters, intravenous fluid administration systems, and similar medical devices which require prolonged patient contact and thus present significant infection risks. Contamination may result from the patients' own flora or from healthcare workers' hands during insertion, and/or manipulation of the device, or from both the patient and the healthcare worker. Medical devices coated with antimicrobial materials can reduce the transfer of such microbes to patients, thereby improving the safety and efficacy of the these devices. Such antimicrobial coatings often include silver metal or silver salts, or other metals with demonstrable antimicrobial activity such as copper, gold, zinc, cerium, platinum, palladium, or tin.

Silver and salts thereof are commonly used because of their demonstrated broad spectrum antimicrobial activity against various bacteria, viruses, yeast, fungi, and protozoa. It is theorized that the observed antimicrobial activity is primarily due to the ability of silver ions to tightly bind nucleophilic functional groups containing sulfur, oxygen or nitrogen. Many nucleophilic functional groups such as thiols, carboxylates, phosphates, alcohols, amines, imidazoles, and indoles are prevalent in biomolecules. Upon binding of ionized silver to these various nucleophilic functional groups, it is believed that widespread disruption and inactivation of microbial biomolecules (and thus antimicrobial activity) occurs.

Silver and salts thereof have therefore been used as antimicrobial agents in a wide variety of applications; for example, they have been incorporated in the absorbent materials of wound care products such as dressings, gels, and bandages, and also in compositions for providing antimicrobial coatings on medical devices. Polymeric binders frequently are added to such silver- or silver salt-containing compositions in order to facilitate manufacturing and/or deposition. One disadvantage frequently observed with such antimicrobial compositions, however, involves relatively poor silver ion elution. Many polymer binder-containing silver or silver salt compositions also can exhibit unsatisfactory antimicrobial efficacy profiles. Various factors can contribute to undesirable efficacy profiles, such as nonuniform thickness of the coating. One disadvantage of some metallic silver-containing antimicrobial coatings is their color/opaqueness, which prevents a healthcare provider from being able to see through the medical device substrate. Thin film coatings comprising silver, for example, can be brown in color. Thus, when such colored silver films are applied to transparent surfaces, the coated surfaces typically have a brown color and significantly diminished transparency.

In contrast to coatings comprising metallic silver, many coatings comprising silver salts are transparent or translucent, and/or lack a colored appearance. Thus, when silver salt coatings are applied to transparent surfaces, the coated surfaces typically have little color and are highly transparent. While coatings comprising silver salts are often translucent, it is extremely difficult to solubilize such compounds and thus to directly deposit coatings comprising silver salts.

The US patent No 6,106,505 discloses an antimicrobial coating containing chlorhexidine diacetate and silver sulfadiazine. The US patent No 6,113,636 describes a method for preparing an antimicrobial coating comprising the precipitation of silver chloride followed by the light induced partial reduction of silver ions to silver metal.

### SUMMARY

The present disclosure is directed to a method for forming an antimicrobial coating on a substrate surface according to claim 1. The methods include providing a mixture comprising a transition metal, a biguanide compound, and a reducing agent; and depositing the mixture onto a substrate surface, thereby forming a coated substrate surface. The mixture is free of polymeric binders.

The substrate surfaces can comprise plastic, glass, metal, or mixtures or laminates thereof. The substrate surfaces can comprise surfaces of medical devices or medical device components. Preferred examples of substrate surfaces include polycarbonate medical devices. The substrate surface also can comprise surfaces of medical fluid containers or medical fluid flow systems. Preferred examples of medical fluid flow systems include I.V. sets and components thereof, such as luer access devices.

The transition metals can comprise various metals or mixtures of metals. Preferred metals include silver, copper, gold, zinc, cerium, platinum, palladium, and tin.

The present invention also provides a coating according to claim 15.

Also disclosed is a coating composition comprising an aqueous solution containing a reducing agent and a complex comprising ionic silver and chlorhexidine, wherein the solution is free of polymeric binders.

### DETAILED DESCRIPTION

The present disclosure is directed to methods for forming an antimicrobial coating on a substrate surface. The methods according to the disclosure involve providing a mixture comprising a transition metal, a biguanide compound, and a reducing agent; and depositing the mixture onto a substrate surface, thereby forming a coated substrate surface. The mixture is free of polymeric binders.

The substrate surfaces of the present disclosure can comprise various materials including, for example, glasses, metals, plastics, ceramics, and elastomers, as well as mixtures and/or laminates thereof. Suitable examples of plastics include acrylonitrile butadiene styrenes, polyacrylonitriles, polyamides, polycarbonates, polyesters, polyetheretherketones, polyetherimides, polyethylenes such as high density polyethylenes and low density polyethylenes, polyethylene terephthalates, polylactic acids, polymethyl methyacrylates, polypropylenes, polystyrenes, polyurethanes, poly(vinyl chlorides), polyvinylidene chlorides, polyethers, polysulfones, silicones, and blends and copolymers thereof. Suitable elastomers include, but are not limited to, natural rubbers, and synthetic rubbers, such as styrene butadiene rubbers, ethylene propylene diene monomer rubbers (EPDM), polychloroprene rubbers (CR), acrylonitrile butadiene rubbers (NBR), chlorosuphonated polyethylene rubbers (CSM), polyisoprene rubbers, isobutylene-isoprene copolymeric rubbers, chlorinated isobutylene-isoprene copolymeric rubbers, brominated isobutylene-isoprene copolymeric rubbers, and blends and copolymers thereof.

In one preferred embodiment of the present disclosure, the antimicrobial coating is formed on (or applied to) a surface of a medical device or medical device component. Medical devices and medical device components which can benefit from the methods according to the disclosure, include, but are not limited to, instruments, apparatuses, implements, machines, contrivances, implants, and components and accessories thereof, intended for use in the diagnosis, cure, mitigation, treatment, or prevention of disease or other condition in humans or other animals, or intended to affect the structure or any function of the body of humans or other animals. Such medical devices are described, for example, in the official National Formulary, the United States Pharmacopoeia, and any supplements thereto. Representative medical devices include, but are not limited to: catheters, such as venous catheters, urinary catheters, Foley catheters, and pain management catheters; stents; abdominal plugs; feeding tubes; cotton gauzes; wound dressings; contact lenses; lens cases; bandages; sutures; hernia meshes; mesh-based wound coverings, implants, metal screws, and metal plates. Additional exemplary medical devices include, but are not limited to, medical fluid containers, medical fluid flow systems, and medical devices such as stethoscopes which regularly come into contact with a patient. One example of a medical fluid flow system is an intravenous fluid administration set, also known as an I.V. set, used for the intravenous administration of fluids to a patient. A typical I.V. set uses plastic tubing to connect a phlebotomized subject to one or more medical fluid sources, such as intravenous solutions or medicament containers. I.V. sets optionally include one or more access devices providing access to the fluid flow path to allow fluid to be added to or withdrawn from the IV tubing. Access devices advantageously eliminate the need to repeatedly phlebotomize the subject and allow for immediate administration of medication or other fluids to the subject, as is well known. Access devices can be designed for use with connecting apparatus employing standard luers, and such devices are commonly referred to as "luer access devices," "luer-activated devices," or "LADs." LADs can be modified with one or more features such as antiseptic indicating devices. Various LADs are illustrated in U.S. Pat. Nos. 6,682,509, 6,669,681, 6,039,302, 5,782,816, 5,730,41.8, 5,360,413, and 5,242,432, and U.S. Patent Application Publication Nos. 2003/0208165, 2003/0141477, 2008/0021381, and 2008/0021392.

I.V. sets can incorporate additional optional components including, for example, septa, stoppers, stopcocks, connectors, adaptors, clamps, extension sets, filters, and the like. Thus, suitable medical devices and medical device components which may be processed in accordance with the methods of the present disclosure include, but are not limited to: I.V. tubing, I.V. fluid bags, I.V. set access devices, septa, stopcocks, I.V. set connectors, I.V. set adaptors, clamps, I.V. filters, catheters, needles, stethoscopes, and cannulae. Representative access devices include, but are not limited to: luer access devices and needleless luer access devices.

The surface of the medical device or medical device component can be fully or partially coated with the antimicrobial coating. The coating can be formed on (or applied to) an exterior surface of the device (i.e., a surface which is intended to come into contact with a patient or healthcare provider), an interior surface of the device (i.e. a surface which is not intended to come into contact with a patient or healthcare provider, but which can come into contact with the patient's blood or other fluids), or both. Suitable medical devices and medical device components are illustrated in U.S. Pat. Nos. 4,412,834, 4,417,890, 4,440,207, 4,457,749, 4,485,064, 4,592,920, 4,603,152, 4,738,668, 5,630,804, 5,928,174, 5,948,385, 6,355,858, 6,592,814, 6,605,751, 6,780,332, 6,800,278, 6,849,214, 6,878,757, 6,897,349. 6,921,390, and 6,984,392, and U.S. Patent Application Publication No. 2007/0085036.

### Antimicrobial Coatings

The coatings of the present disclosure comprise a transition metal or mixtures of transition metals. The transition metals are typically selected to have antimicrobial properties. Suitable metals for use in the compositions include, but are not limited to: silver, copper, gold, zinc, cerium, platinum, palladium, and tin. Coatings comprising a combination of two or more of the foregoing metals can also be used.

In one embodiment, the transition metal is provided as a water-soluble metal salt. Suitable water soluble metal salts have a solubility product (Kₛₚ) greater than 10⁻⁸, for example, greater than 10⁻⁶, greater than 10⁻⁴, and/or greater than 10⁻². Suitable metal salts include, but are not limited to: metal sulfadiazines, metal acetates, metal sulfates, metal nitrates, metal chlorates, metal bromates, metal iodates, and mixtures of the foregoing.

Exemplary metal salts include, but are not limited to, silver salts, such as silver sulfadiazine, silver acetates, silver sulfates, silver nitrates, silver chlorates, silver bromates, silver iodates, and mixtures, of the foregoing.

In another embodiment, the transition metal is provided as a metal biguanide complex. Suitable metal biguanide complexes include, but are not limited to: metal chlorhexidine complexes, metal carbamimidoyl guanidine complexes, metal metformin complexes, metal buformin complexes, metal phenformin complexes, and mixtures and derivatives thereof. Exemplary metal biguanide complexes include, but are not limited to: silver chlorhexidine complexes, silver carbamimidoyl guanidine complexes, silver metformin complexes, silver buformin complexes, silver phenformin complexes, and mixtures and derivatives thereof.

The transition metals in accordance with the present disclosure can comprise particles, such as microparticles or nanoparticles. The metal particles typically have a diameter in the range of 1 nanometer to 50 micrometers, for example, from 10 nanometers to 25 micrometers, from 50 nanometers to 10 micrometers, and/or from 100 nm to 1 micrometer.

In accordance with the methods of the present disclosure, the coatings comprise a biguanide compound. Suitable biguanide compounds include, but are not limited to chlorhexidine, chlorhexidine salts, carbamimidoyl guanidines, metformin, buformin, phenformin, and mixtures and derivatives thereof. Exemplary chlorhexidine salts include chlorhexidine acetates, chlorhexidine gluconates, chlorhexidine hydrochlorides, chlorhexidine sulfates, and mixtures of the foregoing.

Beneficially, the transition metal and the biguanide compound can be selected to have a synergistic effect. One preferred combination comprises silver and chlorhexidine.

In accordance with the methods of the present disclosure, the coatings comprise a reducing agent. It is theorized that the reducing agent facilitates deposition of the coating on the substrate surface by reducing the transition metal, while the reducing agent itself becomes oxidized. The methods disclosed herein further comprise partially reducing the transition metal. Partial reduction of the metal can be carried out by preventing the reduction reaction from reaching completion, for example, by removing the substrate from the reaction mixture after a period of time shorter than the period of time necessary for the reaction to reach completion or by providing a sub-stoichiometric amount of the reducing agent relative to the metal. Partial reduction of the metal can be beneficial, as this allows the coating surface to comprise a mixture of metals having different oxidation states, including both fully reduced and non-reduced metals. Silver coatings, for example, can comprise a mixture of Ag(0) and Ag(I). Metals having different oxidation states may have different efficacies against different pathogens, and/or different elution profiles. Further, by only conducting partial reduction, a certain amount of the transition metal remains in the bulk composition whereas a majority of the metal, when fully reduced, is deposited at the surface of the coating. Such coatings comprising less fully reduced metal at the surface of the coating can display improved elution profiles and/or increased efficacy.

Various reducing agents can be used provided the reducing agent has a sufficient reduction potential to partially reduce the metal of the coating. Suitable reducing agents for use in the disclosed methods include, but are not limited to, aldehydes and alcohols such as, acyclic aliphatic aldehydes, cyclic aliphatic aldehydes, aryl aldehydes, aldoses, acyclic aliphatic alcohols, cyclic aliphatic alcohols, aryl alcohols, ketoses, and mixtures of the foregoing. Exemplary aldehydes include glyceraldehyde, erythrose, threose, ribose, arabinose, xylose, lyxose, allose, altrose, glucose, mannose, gulose, idose, galactose, talose, formaldehyde, and mixtures of the foregoing. Exemplary alcohols include dihydroxyacetone, erythrulose, ribulose, xylulose, fructose, psicose, sorbose, tagatose, methanol, ethanol, benzyl alcohol, and mixtures of the foregoing.

The antimicrobial coatings in accordance with the present disclosure are free of polymeric binders. The antimicrobial coating formulations, however, can comprise one or more additives. Suitable additives include, but are not limited to: adhesion promoters, such as silane adhesion promoters and N-vinyl pyrrolidone; and cationic, anionic, non-ionic, and zwitterionic surfactants, such as lipids, fatty acid salts (e.g., sodium laureate, potassium linoleate, potassium stearate), quaternary ammonium compounds (e.g., hexadecyl trimethyl ammonium bromide), polyethoxylated tallow amines, benzethonium chloride, polysorbates, PLURONIC® copolymers, and sulfates (e.g., potassium hexadecyl phenyl ether sulfonate, potassium resorcinol dioctyl ether sulfonate, and sodium dioctylsulfosuccinate).

The antimicrobial coatings of the present disclosure are formed by providing a mixture comprising one or more transition metals, one or more biguanide compounds, and one or more reducing agents; and depositing the mixture onto a substrate surface, thereby forming a coated substrate surface. Depositing the mixture onto the substrate surface can be carried out by various means, for example, soaking, dipping, and/or swabbing. Optionally, depositing the mixture onto the substrate surface comprises heating the mixture, for example, to a temperature of 40 °C to 80°C.

The disclosure also is directed to a coating composition comprising an aqueous solution containing a reducing agent and a complex comprising ionic silver and chlorhexidine.

### Processing Methods

The antimicrobial coatings of the present disclosure can be further processed by exposing the previously formed coating to a mixture comprising an oxidizing agent and an anion. As previously discussed, many metallic coatings are opaque, or exhibit a colored appearance. Some silver coatings, for example, are brown in color, and thus substrates carrying such coatings typically have a brown color and exhibit poor transparency. Exposing such substrate surfaces to a mixture of an oxidizing agent and an anion according to the methods disclosed herein can advantageously increase the transparency of the metal coating, thereby providing, for example, an efficient method for obtaining medical devices comprising a more transparent antimicrobial coating. Accordingly, the disclosed methods can advantageously increase the transparency of such coatings and hence the transparency of substrate surfaces carrying such coatings.

In contrast to coatings comprising metals, many coatings comprising metal salts are transparent or translucent, and/or lack a colored appearance. Thus, substrates carrying such coatings typically are clear or have a light color, and can be highly transparent. Exposing substrate surfaces carrying metal coatings to a mixture of an oxidizing agent and an anion according to the methods disclosed herein is envisioned to form metal salts comprising an oxidized form of the metal complexed with the anion as a counterion. Accordingly, it is believed the disclosed methods can advantageously form metal salts in the coatings, thereby increasing the transparency of such coatings and hence the transparency of substrate surfaces carrying such coatings.

The antimicrobial activity and the optical properties of coatings processed according to the methods disclosed herein can be affected by various chemical properties of the coatings, such as the incorporation of the anion in the coatings, the formation of metal salts comprising an oxidized form of the metal complexed with the anion as a counterion, and other factors. Exposing a substrate surface carrying a coating comprising a metal to a mixture of an oxidizing agent and an anion according to the methods disclosed herein can alter the chemical properties of the coating, for example, by causing formation of metal salts, thereby providing nanoparticle coatings having increased antimicrobial efficacy and/or improved optical properties.

The oxidizing agents of the present disclosure include a wide variety of known agents for oxidizing metals. Suitable oxidizing agents include metal ions and metal-containing compounds, such as Fe³⁺, Fe²⁺, Cu²⁺, Cu⁺, MnO₄⁻, and Cu⁴⁺; halogens and halogen-containing compounds, such as IO₃⁻, I₃⁻, I₂, BrO₃⁻, Br₂, Br₃⁻, ClO₃⁻ and Cl₂; inorganic and organic compounds of oxygen, such as NO₃⁻, O₂, S₂O₈²⁻. H₂O₂, quinones, and fumarate; and methylene blue. Mixtures of oxidizing agents also are include. It should be understood that any known oxidizing agent could be used provided it has a sufficient oxidation potential to at least partially oxidize the metal included in the coating. Various concentrations of the oxidizing agent can be used, and these oxidizing agent concentrations can be readily determined by one of ordinary skill. Typical amounts of oxidizing agent can range from 0.0001 M to 5 M, for example, 0.001 M to 5 M, 0.01 M to 2.5 M, 0.05 M to 1 M, and/or 0.1 M to 0.5 M, but higher and lower concentrations of oxidizing agents also can be used.

The anions of the present disclosure include a wide variety of known anions, including organic and inorganic anions. Suitable anions include carboxylates, such as acetate, citrate, deoxycholate, fatty acid anions (e.g., decanoate, laurate, myristate, palmitate, stearate, eicosanoate, docsanoate, tetracosanoate, α-linolenate, stearidonate, eicosapentaenoate, docosahexaenoate, linoleate, γ-linolenate, dihomo-γ-linolenate, arachidonate, oleate, erucate, and nervonate), succinate, anionic carboxymethylcellulose, and alginate; halides, such as, fluoride, chloride, bromide, and iodide; halogen-containing anionic compounds, such as chlorate, bromate, and iodate; organic and inorganic oxyanions such as hydroxide, carbonate, oxalate, phosphates, pyrophosphates, phosphonates, phospholipids, sulfates, sulfonates, and cyanate; nitrogen anions, such as amide anions, sulfadiazine anions, cyanates, cyanides. Mixtures of anions may also be used. Various concentrations of the anion can be used, and these anion concentration can be readily determined by one of ordinary skill. Typical amounts of anion can range from 0.0001 M to 10 M, for example, 0.001 M to 7 M, 0.01 M to 5M, 0.05 M to 2.5 M, and/or 0.1 M to 1 M, but higher and lower concentrations of anions also can be used.

In one embodiment, the oxidizing agent and the anion of the present disclosure can be the same. Examples of such "dual oxidizing agents/anions" include chlorate (ClO₃⁻), bromate (BrO₃⁻), and iodate (IO₃⁻). The oxidizing agent and/or the anion also can be generated *in situ*, for example, by dissolution of a salt in a solution, by protonation or deprotonation, or by a reaction that produces the oxidizing agent and/or anion. For example. FeCl₃ can dissolve in aqueous solution to form Fe³⁺ as an oxidizing agent and Cl⁻ as an anion, or I₂ can react in aqueous solution to form H₂OI⁺ and iodide (I⁻) as an anion. An equilibrium reaction also can generate the oxidizing agent and/or the anion.

In one embodiment of the present disclosure, the exposing to the mixture comprising an oxidizing agent and an anion comprises exposing the substrate surface to povidone iodine. Povidone iodine comprises a complex of molecular iodine (I₂) with polyvinyl pyrrolidone (PVP). Molecular iodine is a known oxidizing agent, and as discussed above, the iodide anion can be obtained in aqueous solution, for example, from I₂.

The substrate surfaces of the present disclosure can be exposed to the mixture comprising the oxidizing agent and anion by various known methods. Typical methods for exposing the substrate surface to the mixture comprising the oxidizing agent and anion include dipping, immersing, soaking, submerging, swabbing, spraying, washing, or otherwise contacting the substrate surface with the mixture comprising the oxidizing agent and the anion. The substrate surfaces can be exposed to the mixture comprising the oxidizing agent and anion for various periods of time. The length of desired exposure can be readily determined by one of ordinary skill, and can vary depending on the reactivity of the mixture comprising the oxidizing agent and the anion and/or the desired properties of the final coating composition. Typically, the substrate surface is exposed for 0.1 seconds to 24 hours, but shorter and longer exposure periods can be used. Generally, the substrate surface is exposed to the mixture of the oxidizing agent and anion for 0.1 seconds to 2 hours, 0.5 seconds to 1 hour, 1 second to 30 minutes, and/or 1 minute to 10 minutes. The substrate surfaces also can be sequentially exposed to more than one mixture comprising an oxidizing agent and an anion, the second mixture of which may be the same as or different from the first mixture.

The disclosure may be better understood by reference to the following examples which are not intended to be limiting, but only exemplary of specific embodiments of the disclosure.

### EXAMPLES

### Example 1

### Preparation of Antimicrobial Coatings on Polycarbonate Surfaces

To a 0.1 N solution of AgNO₃ (10 g, available from VWR International) was added chlorhexidine (0.1 g, available from Sigma-Aldrich Co.). A polycarbonate substrate was submerged in the resulting solution and D-(+)-glucose (0.5 g, available from Sigma-Aldrich Co.) was added. The reaction was heated at 60 °C for 1 hour, after which time a brown coating had been deposited on the polycarbonate substrate surface.

### Example 2

The antimicrobial activity of coated surface prepared according to the procedure in Example 1 was tested against *Staphylococcus aureus* (*S. aureus*). A suspension of *S*. *aureus* was grown in tryptic soy broth for 18-24 hours. The suspension was then diluted in saline to 2.63 x 10⁵ colony-forming untits per mL (cfu/mL). Tubes containing 5 mL saline were inoculated with 0.1 mL (2.63 x 10⁴ cfu) of the suspension. The coated surfaces were aseptically added to the tubes, which were incubated at 23 °C for 48 hours. The samples then were plated in tryptic soy agar in triplicate and incubated at 23 °C for 48 hours. After this time, growth of *S. aureus* was measured, as shown in Table 1.

**Table 1**

| **Sample** | **Sample 1 Recovery (cfu)** | **Sample 2 Recovery (cfu)** | **Sample 3 Recovery (cfu)** | **Average (cfu)** | **log (Average)** |
|---|---|---|---|---|---|
| Uncoated control | 9.9 x 10⁴ | 2.2 x 10⁵ | 1.04 x 10⁵ | 1.4 x 10⁵ | 5.15 |
| Coated surface | 2.1 x 10² | 9.9 x 10² | 1.4 x 10² | 4.4 x 10² | 2.65 |

The coating comprising silver and chlorhexidine demonstrated antimicrobial activity against *S*. *aureus,* as determined by a comparison of *S*. *aureus* recovery from a coated substrate to *S*. *aureus* recovery from a substrate lacking a silver/chlorhexidine coating. In particular, the silver coatings prepared accorded to the disclosed methods showed greater than a 300-fold reduction in *S*. *aureus* growth compared to an uncoated surface.

## Claims

1. A method for forming an antimicrobial coating on a substrate surface comprising:
providing a mixture comprising a transition metal, a biguanide compound, and a reducing agent, wherein the mixture is free of polymeric binders and the reducing agent has a sufficient reduction potential to reduce the transition metal; and
depositing the mixture onto a substrate surface, thereby forming the antimicrobial coating on the substrate surface, wherein the transition metal is partially reduced such that the transition metal of the coating has different oxidation states.

2. The method of claim 1, wherein the substrate surface comprises at least one plastic, glass, metal, ceramic, elastomer, or mixtures or laminates thereof.

3. The method of claim 1, wherein the substrate surface comprises a surface of a medical device or medical device component, preferably a surface of an I.V. set.

4. The method of claim 1, wherein the transition metal comprises a metal selected from the group consisting of silver, copper, gold, zinc, cerium, platinum, palladium, tin, and mixtures thereof, preferably silver.

5. The method of claim 1, wherein the transition metal is provided as a water-soluble metal salt.

6. The method of claim 1, wherein the transition metal and the biguanide compound are provided as a metal biguanide complex.

7. The method of claim 5, wherein the metal salt is selected from the group consisting of: metal acetates, metal sulfates, metal nitrates, metal chlorates, metal bromates, metal iodates, and mixtures thereof, preferably wherein the metal salt comprises silver nitrate.

8. The method of claim 1, wherein the transition metal comprises particles having a diameter of 1 nanometer to 50 micrometers.

9. The method of claim 1, wherein the biguanide compound comprises chlorhexidine or salts thereof.

10. The method of claim 1, wherein the biguanide compound comprises a compound selected from the group consisting of chlorhexidine acetates, chlorhexidine gluconates, chlorhexidine hydrochlorides, chlorhexidine sulfates, carbamimidoyl guanidines, metformin, buformin, phenformin, and mixtures and derivatives thereof.

11. The method of claim 1, wherein the reducing agent comprises an aldehyde selected from the group consisting of acyclic aliphatic aldehydes, cyclic aliphatic aldehydes, aryl aldehydes, aldoses, and mixtures thereof.

12. The method of claim 1, wherein the reducing agent comprises an aldehyde selected from the group consisting of glyceraldehyde, erythrose, threose, ribose, arabinose, xylose, lyxose, allose, altrose, glucose, mannose, gulose, idose, galactose, talose, and mixtures thereof.

13. The method of claim 1, wherein the depositing comprises heating the mixture to a temperature of 40 °C to 80 °C.

14. The method of claim 1, wherein the transition metal comprises ionic silver and the biguanide compound comprises chlorhexidine.

15. A coating comprising:
a complex comprising ionic silver and chlorhexidine, wherein the coating is free of polymeric binders and the coating comprises a mixture of Ag(0) and Ag(I).

## Patentansprüche

1. Verfahren zur Erzeugung einer antimikrobiellen Beschichtung auf einer Substratoberfläche, umfassend:
Bereitstellen eines Gemisches, umfassend ein Übergangsmetall, eine Biguanidverbindung und ein Reduktionsmittel, wobei das Gemisch frei von polymeren Bindemitteln ist und das Reduktionsmittel ein ausreichendes Reduktionspotential aufweist, um das Übergangsmetall zu reduzieren, und
Abscheiden des Gemisches auf eine Substratoberfläche, wodurch die antimikrobielle Beschichtung auf der Substratoberfläche erzeugt wird, wobei das Übergangsmetall teilweise reduziert wird, so dass das Übergangsmetall der Beschichtung unterschiedliche Oxidationszustände aufweist.

2. Verfahren nach Anspruch 1, wobei die Substratoberfläche mindestens einen Kunststoff, Glas, Metall, Keramik, Elastomer oder Gemische oder Laminate davon umfaßt.

3. Verfahren nach Anspruch 1, wobei die Substratoberfläche eine Oberfläche einer medizinischen Vorrichtung oder eines Bestandteils einer medizinischen Vorrichtung, vorzugsweise eine Oberfläche eines I.V.-Sets, umfaßt.

4. Verfahren nach Anspruch 1, wobei das Übergangsmetall ein Metall, ausgewählt aus der Gruppe, bestehend aus Silber, Kupfer, Gold, Zink, Cer, Platin, Palladium, Zinn und Gemische davon, vorzugsweise Silber, umfaßt.

5. Verfahren nach Anspruch 1, wobei das Übergangsmetall als ein wasserlösliches Metallsalz bereitgestellt wird.

6. Verfahren nach Anspruch 1, wobei das Übergangsmetall und die Biguanidverbindung als ein Metall-Biguanid-Komplex bereitgestellt werden.

7. Verfahren nach Anspruch 5, wobei das Metallsalz aus der Gruppe, bestehend aus Metallacetaten, Metallsulfaten, Metallnitraten, Metallchloraten, Metallbromaten, Metalliodaten und Gemischen davon, ausgewählt ist, wobei das Metallsalz vorzugsweise Silbernitrat umfaßt.

8. Verfahren nach Anspruch 1, wobei das Übergangsmetall Teilchen umfaßt, die einen Durchmesser von 1 Nanometer bis 50 Mikrometer aufweisen.

9. Verfahren nach Anspruch 1, wobei die Biguanidverbindung Chlorhexidin oder Salze davon umfaßt.

10. Verfahren nach Anspruch 1, wobei die Biguanidverbindung eine Verbindung, ausgewählt aus der Gruppe, bestehend aus Chlorhexidinacetaten, Chlorhexidinglyconaten, Chlorhexidinhydrochloriden, Chlorhexidinsulfaten, Carbamimidoylguanidinen, Metformin, Buformin, Phenformin und Gemischen und Derivaten davon, umfaßt.

11. Verfahren nach Anspruch 1, wobei das Reduktionsmittel ein Aldehyd, ausgewählt aus der Gruppe, bestehend aus acyclischen aliphatischen Aldehyden, cyclischen aliphatischen Aldehyden, Arylaldehyden, Aldosen und Mischungen davon, umfaßt.

12. Verfahren nach Anspruch 1, wobei das Reduktionsmittel ein Aldehyd, ausgewählt aus der Gruppe, bestehend aus Glycerinaldehyd, Erythrose, Threose, Ribose, Arabinose, Xylose, Lyxose, Allose, Altrose, Glucose, Mannose, Gulose, Idose, Galactose, Talose und Gemischen davon, umfaßt.

13. Verfahren nach Anspruch 1, wobei das Abscheiden das Erwärmen des Gemisches auf eine Temperatur von 70°C bis 80°C umfaßt.

14. Verfahren nach Anspruch 1, wobei das Übergangsmetall ionisches Silber, und die Biguanidverbindung Chlorhexidin umfaßt.

15. Beschichtung, umfassend:
einen Komplex, umfassend ionisches Silber und Chlorhexidin, wobei die Beschichtung frei von polymeren Bindemitteln ist, und die Beschichtung ein Gemisch von Ag(0) und Ag(I) umfaßt.

## Revendications

1. Procédé de formation d'un revêtement antimicrobien sur une surface de substrat, comprenant :
la fourniture d'un mélange comprenant un métal de transition, un composé biguanide et un agent réducteur, dans lequel le mélange est dépourvu de liants polymériques et l'agent réducteur a un potentiel de réduction suffisant pour réduire le métal de transition ; et
le dépôt du mélange sur une surface de substrat, formant ainsi le revêtement antimicrobien sur la surface du substrat, dans lequel le métal de transition est partiellement réduit de sorte que le métal de transition sur le revêtement ait des états d'oxydation différents.

2. Procédé selon la revendication 1, dans lequel la surface du substrat comprend au moins un composant parmi le plastique, le verre, le métal, la céramique, un élastomère ou des mélanges ou stratifiés de ceux-ci.

3. Procédé selon la revendication 1, dans lequel la surface du substrat comprend une surface d'un dispositif médical ou un composant de dispositif médical, de préférence une surface d'un ensemble I.V.

4. Procédé selon la revendication 1, dans lequel le métal de transition comprend un métal choisi dans le groupe comprenant l'argent, le cuivre, l'or, le zinc, le cérium, le platine, le palladium, l'étain et leurs mélanges, de préférence, l'argent.

5. Procédé selon la revendication 1, dans lequel le métal de transition est fourni en tant que sel de métal soluble dans l'eau.

6. Procédé selon la revendication 1, dans lequel le métal de transition et le composé biguanide sont fournis en tant que complexe de métal biguanide.

7. Procédé selon la revendication 5, dans lequel le sel métallique est choisi dans le groupe comprenant des acétates de métal, des sulfates de métal, des nitrates de métal, des chlorates de métal, des bromates de métal, des iodates de métal et leurs mélanges, de préférence dans lequel le sel de métal comprend le nitrate d'argent.

8. Procédé selon la revendication 1, dans lequel le métal de transition comprend des particules ayant un diamètre de 1 nanomètre à 50 micromètres.

9. Procédé selon la revendication 1, dans lequel le composé biguanide comprend de la chlorhexidine ou des sels de celle-ci.

10. Procédé selon la revendication 1, dans lequel le composé biguanide comprend un composé choisi dans le groupe comprenant les acétates de chlorhexidine, les gluconates de chlorhexidine, les chlorhydrates de chlorhexidine, les sulfates de chlorhexidine, les carbaminidoyl-guanidines, la metformine, la butformine, la phenformine et leurs mélanges et dérivés.

11. Procédé selon la revendication 1, dans lequel l'agent réducteur comprend un aldéhyde choisi dans le groupe comprenant des aldéhydes acycliques aliphatiques, des aldéhydes cycliques aliphatiques, des arylaldéhydes, des aldoses et leurs mélanges.

12. Procédé selon la revendication 1, dans lequel l'agent réducteur comprend un aldéhyde choisi dans le groupe comprenant le glycéraldéhyde, l'érythrose, le thréose, le ribose, l'arabinose, le xylose, le lyxose, l'allose, l'altrose, le glucose, le mamnose, le gulose, l'idose, le galactose, le talose et leurs mélanges.

13. Procédé selon la revendication 1, dans lequel le dépôt comprend le chauffage du mélange à une température de 40° C à 80° C.

14. Procédé selon la revendication 1, dans lequel le métal de transition comprend l'argent ionique et le composé biguanide comprend la chlorhexidine.

15. Revêtement comprenant :
un complexe comprenant de l'argent ionique et de la chlorhexidine, le revêtement étant dépourvu de liants polymères et le revêtement comprenant un mélange d'Ag(0) et d'Ag(1).
